# EUROPEAN PATENT APPLICATION

(11) **EP 4 736 876 A1**
(43) Date of publication of application: **06.05.2026**
(21) Application number: 24830975.9
(22) Date of filing: 28.06.2024
(51) Int. Cl.: A61K 45/06, A61K 31/198, A61K 31/40, A61P 35/00

(54) **DRUG COMBINATION CONTAINING PD-L1 SMALL MOLECULE INHIBITOR AND USE THEREOF**

(30) Priority: 29.06.2023 CN 202310785638; 20.12.2023 CN 202311762592
(71) Applicant: Beijing Maxinovel Pharmaceuticals Co., Ltd., Beijing 100176 (CN)
(72) Inventor: WANG, Yuguang, Guangzhou, Guangdong 510670 (CN); FENG, Zhenhua, Guangzhou, Guangdong 510670 (CN); ZHAO, Qiang, Guangzhou, Guangdong 510670 (CN)
(74) Representative: Michalski Hüttermann & Partner Patentanwälte mbB
(86) International application number: PCT/CN2024/102360
(87) International publication number: WO 2025/002346

(57) **Abstract**

A drug combination containing a PD-L1 small molecule inhibitor and the use thereof. The drug combination comprises a PD-L1 small molecule inhibitor and a chemotherapeutic drug molecule, or a PD-L1 small molecule inhibitor and a COX-2 inhibitor. The drug combination can be used for treating tumors.

## Description

The present application claims priority to Chinese Patent Application No. 202310785638X filed on June 29, 2023 and Chinese Patent Application No. 2023117625926 filed on December 20, 2023. The contents of the Chinese patent application are incorporated herein by reference in their entirety.

### TECHNICAL FIELD

The present disclosure relates to a drug combination comprising a PD-L1 small molecule inhibitor and the use thereof.

### BACKGROUND

Malignant tumors are major diseases that endanger people's life and health. In recent years, with the rapid development of tumor biology and related disciplines, specific antitumor drugs targeting abnormal signaling systems in tumor cells and immunotherapeutic antitumor drugs have become the focus of new drug research and development. Meanwhile, the combination of multiple antitumor drugs for the treatment of tumor diseases is also a hot topic in scientific research.

Programmed cell death-1 (PD-1), also known as CD279, is a cell surface receptor expressed on activated T cells, natural killer T cells, B cells, and macrophages. It functions as an intrinsic negative feedback system, thereby preventing T-cell activation, which in turn reduces autoimmunity and promotes self-tolerance. Additionally, PD-1 is known to play a significant role in suppressing antigen-specific T-cell responses in diseases such as cancer and viral infections.

The structure of PD-1 consists of an extracellular immunoglobulin variable-like domain, followed by a transmembrane region and an intracellular domain. This intracellular domain contains two phosphorylation sites located within the immunoreceptor tyrosine-based inhibitory motif and the immunoreceptor tyrosine-based switch motif, indicating that PD-1 negatively regulates T-cell receptor-mediated signaling. PD-1 has two ligands, PD-L1 and PD-L2, which differ in their expression patterns. PD-L1 protein is upregulated on macrophages and dendritic cells in response to lipopolysaccharide and GM-CSF treatment and on T cells and B cells following T cell receptor and B cell receptor signaling. PD-L1 is also highly expressed on nearly all tumor cells, and this expression further increases after IFN-γ treatment. A large number of studies have confirmed that PD-L1 on the surface of tumor cells in the tumor microenvironment is increased, and at the same time, PD-L1 binds to PD-1 on activated T cells to transmit negative regulatory signals, leading to apoptosis or immune anergy of tumor antigen-specific T cells, thereby inhibiting the immune response and promoting the escape of tumor cells.

Although no PD-L1 small molecule inhibitors are currently on the market, several are in clinical research stages. Among them, WO2019128918A1 discloses the structure and preparation method of a PD-L1 small molecule inhibitor, with *in vitro* and pharmacokinetic studies demonstrating good antitumor activity. Its specific structure is shown in Formula I.

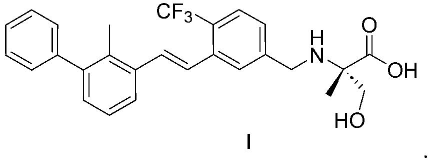

### SUMMARY

The technical problem to be solved by the present disclosure is to overcome the limited variety of antitumor drugs in the prior art and to provide a drug combination comprising a PD-L1 small molecule inhibitor and the use thereof. Compared with the use of PD-L1 small molecule inhibitors alone or other small molecule drugs (e.g., targeted small molecule inhibitors or chemotherapeutic drug molecules), the drug combination of the present disclosure has a synergistic effect, and further has a better effect in treating cancer and prolonging survival time.

The present disclosure provides a drug combination A, comprising a PD-L1 small molecule inhibitor and a chemotherapeutic drug molecule.

In some embodiments, the drug combination A has a synergistic effect.

In the drug combination A, the PD-L1 small molecule inhibitor is preferably a compound of Formula I or a pharmaceutically acceptable salt thereof;

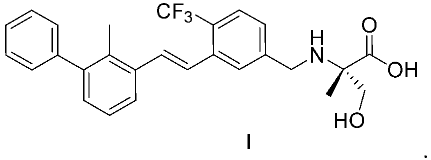

In the drug combination A, the chemotherapeutic drug molecule is preferably one or more of capecitabine, paclitaxel, oxaliplatin, and temozolomide, more preferably capecitabine or paclitaxel.

In some embodiments, the chemotherapeutic drug molecule is temozolomide.

In the drug combination A, the mass ratio of the PD-L1 small molecule inhibitor to the chemotherapeutic drug molecule may be (0.04 to 50):1, preferably (0.2 to 16):1, more preferably (0.2 to 2):1 or (6 to 16):1, for example, 0.45:1, 0.58:1, 1.2:1, 10:1, or 11:1.

In some embodiments, the mass ratio of the PD-L1 small molecule inhibitor to the chemotherapeutic drug molecule is 6:1, 9.5:1, or 16:1.

In some embodiments, the drug combination A comprises the compound of Formula I as described above and capecitabine. The mass ratio of the compound of Formula I to capecitabine is preferably (0.2 to 2):1, for example, 0.45:1, 0.58:1, or 1.2:1.

In some embodiments, the drug combination A comprises the compound of Formula I as described above and paclitaxel. The mass ratio of the compound of Formula I to paclitaxel is preferably (6 to 16):1, for example, 10:1 or 11:1.

In some embodiments, the drug combination A comprises the compound of Formula I as described above and temozolomide. The mass ratio of the compound of Formula I to temozolomide is preferably (6 to 16):1, for example, 6:1, 9.5:1, or 16:1.

The active pharmaceutical ingredients of the drug combination A are preferably the PD-L1 small molecule inhibitor as described above and the chemotherapeutic drug molecule as described above.

In some embodiments, the active pharmaceutical ingredients of the drug combination A are the compound of Formula I as described above and capecitabine.

In some embodiments, the active pharmaceutical ingredients of the drug combination A are the compound of Formula I as described above and paclitaxel.

In some embodiments, the active pharmaceutical ingredients of the drug combination A are the compound of Formula I as described above and temozolomide.

In the drug combination A, the PD-L1 small molecule inhibitor is preferably administered via the gastrointestinal tract (*e.g.,* oral, sublingual, or rectal administration), injection (*e.g.,* subcutaneous, intradermal, intravenous, intraperitoneal, or intramuscular), respiratory administration, or topical administration (*e.g.,* mucosal or cutaneous), more preferably via the gastrointestinal tract, such as oral administration.

In the drug combination A, the chemotherapeutic drug molecule is preferably administered via the gastrointestinal tract (*e.g.,* oral, sublingual, or rectal administration), injection (*e.g.,* subcutaneous, intradermal, intravenous, intraperitoneal, or intramuscular), respiratory administration, or topical administration (*e.g.,* mucosal or cutaneous), preferably via the gastrointestinal tract or injection, such as oral administration or intravenous injection.

In the drug combination A, the PD-L1 small molecule inhibitor is preferably administered orally, and the chemotherapeutic drug molecule is preferably administered orally.

In the drug combination A, the PD-L1 small molecule inhibitor is preferably administered orally, and the chemotherapeutic drug molecule is preferably administered via intravenous injection.

The present disclosure provides a pharmaceutical composition B, comprising a PD-L1 small molecule inhibitor, a chemotherapeutic drug molecule, and a pharmaceutical excipient.

In some embodiments, the pharmaceutical composition B has a synergistic effect.

In the pharmaceutical composition B, the types of the PD-L1 small molecule inhibitor and the chemotherapeutic drug molecule are preferably as described above.

In the pharmaceutical composition B, the content of the PD-L1 small molecule inhibitor is preferably 30 to 500 mg, more preferably 40 to 400 mg, for example, 50 mg, 150 mg, or 300 mg.

In the pharmaceutical composition B, the content of the chemotherapeutic drug molecule is preferably 10 to 650 mg, more preferably 30 to 550 mg, for example, 30 mg, 60 mg, 100 mg, 150 mg, or 500 mg.

In some embodiments, the content of the chemotherapeutic drug molecule is 20 mg.

In the pharmaceutical composition B, the mass ratio of the PD-L1 small molecule inhibitor to the chemotherapeutic drug molecule may be (0.04 to 50):1, preferably (0.2 to 16):1, more preferably (0.2 to 2):1 or (6 to 16):1, for example, 0.45:1, 0.58:1, 1.2:1, 10:1, or 11:1.

In some embodiments, the mass ratio of the PD-L1 small molecule inhibitor to the chemotherapeutic drug molecule is 6:1, 9.5:1, or 16:1.

In some embodiments, the active pharmaceutical ingredients of the pharmaceutical composition B are the PD-L1 small molecule inhibitor as described above and the chemotherapeutic drug molecule as described above, preferably the compound of Formula I and capecitabine, or the compound of Formula I and paclitaxel.

In some embodiments, the active pharmaceutical ingredients of the pharmaceutical composition B are the compound of Formula I and temozolomide.

In some embodiments, when the pharmaceutical composition B comprises the compound of Formula I, capecitabine, and a pharmaceutical excipient, the mass ratio of the compound of Formula I to capecitabine is preferably (0.2 to 2):1, for example, 0.45:1, 0.58:1, or 1.2:1.

In some embodiments, when the pharmaceutical composition B comprises the compound of Formula I, paclitaxel, and a pharmaceutical excipient, the mass ratio of the compound of Formula I to paclitaxel is preferably (6 to 16):1, for example, 10:1 or 11:1.

In some embodiments, when the pharmaceutical composition B comprises the compound of Formula I, temozolomide, and a pharmaceutical excipient, the mass ratio of the compound of Formula I to temozolomide is preferably (6 to 16):1, for example, 6:1, 9.5:1, or 16:1.

The pharmaceutical composition B preferably consists of the PD-L1 small molecule inhibitor, the chemotherapeutic drug molecule, and a pharmaceutical excipient.

The pharmaceutical composition B may be a separate pharmaceutical composition.

The pharmaceutical composition B can be formulated into various suitable dosage forms depending on the route of administration, including gastrointestinal dosage forms (e.g., oral dosage forms) and parenteral dosage forms (*e.g.,* injectable dosage forms, respiratory dosage forms, mucosal dosage forms, or cavity dosage forms), preferably capsules, tablets, pills, granules, powders, or oral liquid preparations.

The pharmaceutical composition B is preferably presented in oral dosage forms or injectable dosage forms.

The present disclosure provides a pharmaceutical composition C, comprising:

a first pharmaceutical composition C-1, comprising a PD-L1 small molecule inhibitor and a pharmaceutical excipient; and,

a second pharmaceutical composition C-2, comprising a chemotherapeutic drug molecule and a pharmaceutical excipient.

In some embodiments, the pharmaceutical composition C has a synergistic effect.

The first pharmaceutical composition C-1 may be a separate pharmaceutical composition, and the second pharmaceutical composition C-2 may be a separate pharmaceutical composition.

In the pharmaceutical composition C, the PD-L1 small molecule inhibitor and the chemotherapeutic drug molecule are as described above.

In the pharmaceutical composition C, the content of the PD-L1 small molecule inhibitor is preferably 30 to 500 mg, more preferably 40 to 400 mg, for example, 50 mg, 150 mg, or 300 mg.

In the pharmaceutical composition C, the content of the chemotherapeutic drug molecule is preferably 10 to 650 mg, more preferably 30 to 550 mg, for example, 30 mg, 60 mg, 100 mg, 150 mg, or 500 mg.

In some embodiments, the content of the chemotherapeutic drug molecule in the pharmaceutical composition C is 20 mg.

In the pharmaceutical composition C, the mass ratio of the PD-L1 small molecule inhibitor to the chemotherapeutic drug molecule is (0.04 to 50):1, preferably (0.2 to 16):1, more preferably (0.2 to 2):1 or (6 to 16):1, for example, 0.45:1, 0.58:1, 1.2:1, 10:1, or 11:1.

In some embodiments, the mass ratio of the PD-L1 small molecule inhibitor to the chemotherapeutic drug molecule in the pharmaceutical composition C is 6:1, 9.5:1, or 16:1.

In some embodiments, the pharmaceutical composition C consists of a first pharmaceutical composition C-1 and a second pharmaceutical composition C-2; The first pharmaceutical composition C-1 consists of the PD-L1 small molecule inhibitor as described above and a pharmaceutical excipient, preferably the compound of Formula I as described above and a pharmaceutical excipient; The second pharmaceutical composition C-2 consists of the chemotherapeutic drug molecule as described above and a pharmaceutical excipient, preferably capecitabine or paclitaxel and a pharmaceutical excipient.

In some embodiments, the pharmaceutical composition C consists of a first pharmaceutical composition C-1 and a second pharmaceutical composition C-2; The first pharmaceutical composition C-1 consists of the compound of Formula I as described above and a pharmaceutical excipient; The second pharmaceutical composition C-2 consists of temozolomide and a pharmaceutical excipient.

In some embodiments, when the first pharmaceutical composition C-1 comprises the compound of Formula I as described above and a pharmaceutical excipient, and the second pharmaceutical composition C-2 comprises capecitabine and a pharmaceutical excipient, then the mass ratio of the compound of Formula I to capecitabine is preferably (0.2 to 2):1, for example, 0.45:1, 0.58:1, or 1.2:1.

In some embodiments, when the first pharmaceutical composition C-1 comprises the compound of Formula I as described above and a pharmaceutical excipient, and the second pharmaceutical composition C-2 comprises paclitaxel and a pharmaceutical excipient, then the mass ratio of the compound of Formula I to paclitaxel is preferably (6 to 16):1, for example, 10:1 or 11:1.

In some embodiments, when the first pharmaceutical composition C-1 comprises the compound of Formula I as described above and a pharmaceutical excipient, and the second pharmaceutical composition C-2 comprises temozolomide and a pharmaceutical excipient, then the mass ratio of the compound of Formula I to temozolomide is preferably (6 to 16):1, for example, 6:1, 9.5:1, or 16:1.

The first pharmaceutical composition C-1 is preferably presented in an oral dosage form.

The second pharmaceutical composition C-2 is preferably presented in an oral dosage form or an injectable dosage form.

The present disclosure provides a combination kit D, comprising:
a first container, comprising the first pharmaceutical composition C-1 as described above; and,
a second container, comprising the second pharmaceutical composition C-2 as described above.

The present disclosure also provides the use of the drug combination A, pharmaceutical composition B, or pharmaceutical composition C as described above in the manufacture of a medicament for treating tumors.

The present disclosure also provides the use of the PD-L1 small molecule inhibitor in combination with the chemotherapeutic drug molecule in the manufacture of a medicament for treating tumors.

The present disclosure also provides the use of the PD-L1 small molecule inhibitor in the manufacture of a medicament for treating tumors, wherein the PD-L1 small molecule is used in combination with the chemotherapeutic drug molecule.

The present disclosure also provides the use of the chemotherapeutic drug molecule in the manufacture of a medicament for treating tumors, wherein the chemotherapeutic drug molecule is used in combination with the PD-L1 small molecule inhibitor.

The present disclosure also provides a method of treating tumors, comprising administering a therapeutically effective amount of the drug combination A, pharmaceutical composition B, or pharmaceutical composition C as described above to a subject in need thereof *(e.g.,* a human).

In the above drug combination A, pharmaceutical composition B, pharmaceutical composition C, the use thereof, and the treatment method thereof:

In some embodiments, the tumor is one or more of gastric cancer, nasopharyngeal carcinoma, lung cancer, colorectal cancer, breast cancer, colon cancer, bladder cancer, cervical cancer, endometrial cancer, pancreatic cancer, prostate cancer, hepatocellular carcinoma, melanoma, head and neck cancer, esophageal cancer, ovarian cancer, bone cancer, central nervous system tumors, adrenal tumors, renal cell carcinoma, and neuroblastoma, preferably breast cancer, gastric cancer, or nasopharyngeal carcinoma.

In some embodiments, the tumor is one or more of gastric cancer, nasopharyngeal carcinoma, lung cancer, colorectal cancer, breast cancer, colon cancer, bladder cancer, cervical cancer, endometrial cancer, pancreatic cancer, prostate cancer, hepatocellular carcinoma, melanoma, head and neck cancer, esophageal cancer, ovarian cancer, bone cancer, central nervous system tumors, adrenal tumors, renal cell carcinoma, neuroblastoma, and glioblastoma, preferably glioblastoma.

In some embodiments, the administration of the PD-L1 small molecule inhibitor and the chemotherapeutic drug molecule may be simultaneous administration or separate administration.

The "simultaneous administration" means that the PD-L1 small molecule inhibitor and the chemotherapeutic drug molecule are comprised in separate pharmaceutical compositions and administered simultaneously; Or, the "separate pharmaceutical composition comprising the PD-L1 small molecule inhibitor" and the "separate pharmaceutical composition comprising the chemotherapeutic drug molecule" are administered simultaneously.

The "separate administration" means that the "separate pharmaceutical composition comprising the PD-L1 small molecule inhibitor" and the "separate pharmaceutical composition comprising the chemotherapeutic drug molecule" are administered at different times, for example: one of the "separate pharmaceutical composition comprising the PD-L1 small molecule inhibitor" and the "separate pharmaceutical composition comprising the chemotherapeutic drug molecule" is administered first, followed by the other. The separate administration may be temporally close or temporally distant.

Regardless of simultaneous or separate administration, the administration regimen of the PD-L1 small molecule inhibitor and the chemotherapeutic drug molecule (including administration route, administration dose, administration interval, *etc.*) may be the same or different, and can be adjusted by those skilled in the art as needed to provide optimal therapeutic effects.

In some embodiments, the total daily dose of the PD-L1 small molecule inhibitor ranges from 30 to 2400 mg; preferably 500 to 2000 mg; for example, 600 mg, 700 mg, 800 mg, 900 mg, 1000 mg, 1100 mg, 1200 mg, 1300 mg, 1400 mg, 1500 mg, 1600 mg, 1700 mg, 1800 mg, or 1900 mg.

In some embodiments, the administration frequency of the PD-L1 small molecule inhibitor is twice daily, once daily, or once every two days, preferably twice daily.

In some embodiments, the total daily dose of the PD-L1 small molecule inhibitor ranges from 500 to 2000 mg, for example, 600 mg, 700 mg, 800 mg, 900 mg, 1000 mg, 1100 mg, 1200 mg, 1300 mg, 1400 mg, 1500 mg, 1600 mg, 1700 mg, 1800 mg, or 1900 mg; The administration frequency is twice daily, once daily, or once every two days, preferably twice daily (BID).

In some embodiments, the total daily dose of the PD-L1 small molecule inhibitor is 1000 mg, 1100 mg, 1200 mg, 1300 mg, 1400 mg, 1500 mg, 1600 mg, 1700 mg, 1800 mg, or 1900 mg; The administration frequency is twice daily.

In some embodiments, the PD-L1 small molecule inhibitor is administered orally at the total daily dose and frequency described above.

In some embodiments, the PD-L1 small molecule inhibitor is administered orally at a total daily dose of 1000 to 1900 mg and a frequency of twice daily.

In some embodiments, the dose of the chemotherapeutic drug molecule is administered based on the subject's body surface area.

In some embodiments, when the chemotherapeutic drug molecule is capecitabine, the total daily dose ranges from 1000 to 3000 mg/m², preferably 1000 mg/m², 1250 mg/m², 1500 mg/m², 1750 mg/m², 2000 mg/m², 2250 mg/m², 2500 mg/m², 2750 mg/m², or 3000 mg/m², for example, 2000 mg/m² or 2500 mg/m²; The administration frequency is once daily or twice daily, preferably twice daily.

In some embodiments, when the chemotherapeutic drug molecule is capecitabine, capecitabine is administered orally at a total daily dose of 1000 to 3000 mg/m² and a frequency of twice daily.

In some embodiments, when the chemotherapeutic drug molecule is paclitaxel, the total daily dose ranges from 50 to 200 mg/m², preferably 50 mg/m², 75 mg/m², 80 mg/m², 100 mg/m², 125 mg/m², 135 mg/m², 145 mg/m², 155 mg/m², 165 mg/m², 175 mg/m², 185 mg/m², 195 mg/m², or 200 mg/m², for example, 80 mg/m², 135 mg/m², or 175 mg/m²; The administration frequency is once every two weeks, once every three weeks, or once every four weeks; preferably once every three weeks.

In some embodiments, when the chemotherapeutic drug molecule is paclitaxel, paclitaxel is administered intravenously at a total daily dose of 50 to 200 mg/m² and a frequency of once every three weeks.

In some embodiments, when the chemotherapeutic drug molecule is temozolomide, the total daily dose ranges from 100 to 250 mg/m², preferably 100 mg/m², 125 mg/m², 150 mg/m², 175 mg/m², 200 mg/m², 225 mg/m², or 250 mg/m², for example, 150 mg/m² or 200 mg/m²; The administration frequency is once daily for 5 days, followed by 23 days off, then entering the next cycle.

In some embodiments, when the chemotherapeutic drug molecule is temozolomide, temozolomide is administered orally at a total daily dose of 100 to 250 mg/m², with an administration frequency of once daily for 5 days, followed by 23 days off, then entering the next cycle.

In some embodiments, the PD-L1 small molecule inhibitor is administered orally at a total daily dose of 1000 to 1900 mg and a frequency of twice daily, and the chemotherapeutic drug molecule is administered orally at a total daily dose of 1000 to 3000 mg/m² and a frequency of twice daily.

In some embodiments, the PD-L1 small molecule inhibitor is administered orally at a total daily dose of 1000 to 1900 mg and a frequency of twice daily, and the chemotherapeutic drug molecule is administered intravenously at a total daily dose of 50 to 200 mg/m² and a frequency of once every three weeks.

In some embodiments, the PD-L1 small molecule inhibitor is administered orally at a total daily dose of 1000 to 1900 mg and a frequency of twice daily, and the chemotherapeutic drug molecule is administered at a total daily dose of 100 to 250 mg/m² and a frequency of once daily for 5 days, followed by 23 days off, then entering the next cycle.

The present disclosure provides a drug combination E, comprising a PD-L1 small molecule inhibitor and a COX-2 inhibitor.

In some embodiments, the drug combination E has a synergistic effect.

In the drug combination E, the PD-L1 small molecule inhibitor is preferably the compound of Formula I or a pharmaceutically acceptable salt thereof;

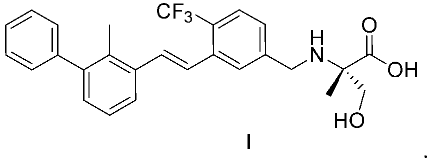

In the drug combination E, the COX-2 inhibitor is preferably nimesulide, meloxicam, celecoxib, or rofecoxib, more preferably celecoxib.

In the drug combination E, the mass ratio of the PD-L1 small molecule inhibitor to the COX-2 inhibitor is preferably (0.1 to 10): 1, more preferably (2 to 10):1, for example, 6: 1 or 7.5:1.

In some embodiments, the active pharmaceutical ingredients of the drug combination E are the PD-L1 small molecule inhibitor as described above and the COX-2 inhibitor as described above, preferably the compound of Formula I as described above and celecoxib.

In the drug combination E, the PD-L1 small molecule inhibitor is preferably administered via the gastrointestinal tract (*e.g.,* oral, sublingual, or rectal administration), injection (*e.g.,* subcutaneous, intradermal, intravenous, intraperitoneal, or intramuscular), respiratory administration, or topical administration (*e.g.*, mucosal or cutaneous), more preferably via the gastrointestinal tract, such as oral administration.

In the drug combination E, the COX-2 inhibitor is preferably administered via the gastrointestinal tract (*e.g.,* oral, sublingual, or rectal administration), injection (*e.g.,* subcutaneous, intradermal, intravenous, intraperitoneal, or intramuscular), respiratory administration, or topical administration (*e.g.,* mucosal or cutaneous), more preferably via the gastrointestinal tract, such as oral administration.

In the drug combination E, the PD-L1 small molecule inhibitor is preferably administered orally, and the COX-2 inhibitor is preferably administered orally.

The present disclosure provides a pharmaceutical composition F, comprising a PD-L1 small molecule inhibitor, a COX-2 inhibitor, and a pharmaceutical excipient.

In some embodiments, the pharmaceutical composition F has a synergistic effect.

In the pharmaceutical composition F, the PD-L1 small molecule inhibitor and COX-2 inhibitor may be as described above.

In the pharmaceutical composition F, the content of the PD-L1 small molecule inhibitor is preferably 30 to 500 mg, more preferably 40 to 400 mg, for example, 50 mg, 150 mg, or 300 mg.

In the pharmaceutical composition F, the content of the COX-2 inhibitor is preferably 50 to 250 mg, more preferably 100 to 200 mg, for example, 100 mg or 200 mg.

In the pharmaceutical composition F, the mass ratio of the PD-L1 small molecule inhibitor to the COX-2 inhibitor is preferably (0.1 to 10): 1, more preferably (2 to 10): 1, for example, 6: 1 or 7.5:1.

In some embodiments, the active pharmaceutical ingredients of the pharmaceutical composition F are the PD-L1 small molecule inhibitor as described above and the COX-2 inhibitor as described above, preferably the compound of Formula I as described above and celecoxib.

In some embodiments, the pharmaceutical composition F consists of the PD-L1 small molecule inhibitor as described above, the COX-2 inhibitor as described above, and a pharmaceutical excipient.

The pharmaceutical composition F may be a separate pharmaceutical composition.

The pharmaceutical composition F can be formulated into various suitable dosage forms depending on the route of administration, including gastrointestinal dosage forms (*e.g.,* oral dosage forms) and parenteral dosage forms (*e.g.,* injectable dosage forms, respiratory dosage forms, mucosal dosage forms, or cavity dosage forms), preferably capsules, tablets, pills, granules, powders, or oral liquid preparations.

The present disclosure also provides a pharmaceutical composition G, comprising:
a first pharmaceutical composition G-1, comprising a PD-L1 small molecule inhibitor and a pharmaceutical excipient; and,
a second pharmaceutical composition G-2, comprising a COX-2 inhibitor and a pharmaceutical excipient.

In some embodiments, the pharmaceutical composition G has a synergistic effect.

In the pharmaceutical composition G, the PD-L1 small molecule inhibitor and COX-2 inhibitor may be as described above.

In some embodiments, the pharmaceutical composition G consists of the first pharmaceutical composition G-1 and the second pharmaceutical composition G-2; The first pharmaceutical composition G-1 consists of the PD-L1 small molecule inhibitor as described above and a pharmaceutical excipient, preferably consisting of the compound of Formula I as described above and a pharmaceutical excipient; The second pharmaceutical composition G-2 consists of the COX-2 inhibitor as described above and a pharmaceutical excipient, preferably consisting of celecoxib and a pharmaceutical excipient.

The first pharmaceutical composition G-1 is a separate pharmaceutical composition; The second pharmaceutical composition G-2 is a separate pharmaceutical composition.

In the pharmaceutical composition G, the content of the PD-L1 small molecule inhibitor is preferably 30 to 500 mg, more preferably 40 to 400 mg, for example, 50 mg, 150 mg, or 300 mg.

In the pharmaceutical composition G, the content of the COX-2 inhibitor is preferably 50 to 250 mg, more preferably 100 to 200 mg, for example, 100 mg or 200 mg.

In the pharmaceutical composition G, the mass ratio of the PD-L1 small molecule inhibitor to the COX-2 inhibitor is preferably (0.1 to 10):1, more preferably (2 to 10):1, for example, 6:1 or 7.5:1.

In the pharmaceutical composition G, the first pharmaceutical composition G-1 is preferably presented in an oral dosage form.

In the pharmaceutical composition G, the second pharmaceutical composition G-2 is preferably presented in an oral dosage form.

In the pharmaceutical composition G, preferably, the first pharmaceutical composition G-1 is presented in an oral dosage form, and the second pharmaceutical composition G-2 is presented in an oral dosage form.

The present disclosure provides a combination kit H, comprising:
a first container, comprising the first pharmaceutical composition G-1 as described above; and,
a second container, comprising the second pharmaceutical composition G-2 as described above.

The present disclosure also provides the use of the drug combination E, pharmaceutical composition F, or pharmaceutical composition G as described above in the manufacture of a medicament for treating tumors.

The present disclosure provides the use of the PD-L1 small molecule inhibitor in combination with the COX-2 inhibitor in the manufacture of a medicament for treating tumors.

The present disclosure provides the use of the PD-L1 small molecule inhibitor in the manufacture of a medicament for treating tumors, wherein the PD-L1 small molecule inhibitor is used in combination with the COX-2 inhibitor.

The present disclosure provides the use of the COX-2 inhibitor in the manufacture of a medicament for treating tumors, wherein the COX-2 inhibitor is used in combination with the PD-L1 small molecule inhibitor.

The present disclosure provides a method of treating tumors, comprising administering a therapeutically effective amount of the drug combination E, pharmaceutical composition F, or pharmaceutical composition G as described above to a subject in need thereof (*e.g.,* a human).

In the above drug combination E, pharmaceutical composition F, pharmaceutical composition G, the use thereof, and the treatment method thereof:

In some embodiments, the tumor is one or more of gastric cancer, nasopharyngeal carcinoma, lung cancer, colorectal cancer, breast cancer, colon cancer, bladder cancer, cervical cancer, endometrial cancer, pancreatic cancer, prostate cancer, hepatocellular carcinoma, melanoma, head and neck cancer, esophageal cancer, ovarian cancer, bone cancer, central nervous system tumors, adrenal tumors, renal cell carcinoma, and neuroblastoma, preferably breast cancer, gastric cancer, or nasopharyngeal carcinoma.

In some embodiments, the tumor is one or more of gastric cancer, nasopharyngeal carcinoma, lung cancer, colorectal cancer, breast cancer, colon cancer, bladder cancer, cervical cancer, endometrial cancer, pancreatic cancer, prostate cancer, hepatocellular carcinoma, melanoma, head and neck cancer, esophageal cancer, ovarian cancer, bone cancer, central nervous system tumors, adrenal tumors, renal cell carcinoma, neuroblastoma, and glioblastoma, preferably glioblastoma.

In some embodiments, the administration of the PD-L1 small molecule inhibitor and the COX-2 inhibitor may be simultaneous administration or separate administration.

The "simultaneous administration" means that the PD-L1 small molecule inhibitor and the COX-2 inhibitor are comprised in separate pharmaceutical compositions and administered simultaneously; Or, the "separate pharmaceutical composition comprising the PD-L1 small molecule inhibitor" and the "separate pharmaceutical composition comprising the COX-2 inhibitor" are administered simultaneously.

The "separate administration" means that the "separate pharmaceutical composition comprising the PD-L1 small molecule inhibitor" and the "separate pharmaceutical composition comprising the COX-2 inhibitor" are administered at different times, for example: one of the "separate pharmaceutical composition comprising the PD-L1 small molecule inhibitor" and the "separate pharmaceutical composition comprising the COX-2 inhibitor" is administered first, followed by the other. The separate administration may be temporally close or temporally distant.

Regardless of simultaneous or separate administration, the administration regimen of the "separate pharmaceutical composition comprising the PD-L1 small molecule inhibitor" and the "separate pharmaceutical composition comprising the COX-2 inhibitor" (including administration route, administration dose, administration interval, *etc*.) may be the same or different, and can be adjusted by those skilled in the art as needed to provide optimal therapeutic effects.

In some embodiments, the PD-L1 small molecule inhibitor may be administered via the gastrointestinal tract (*e.g.,* oral, sublingual, or rectal administration), injection (*e.g.,* subcutaneous, intradermal, intravenous, intraperitoneal, or intramuscular), respiratory administration, or topical administration (*e.g.,* mucosal or cutaneous), preferably via the gastrointestinal tract, such as oral administration.

In some embodiments, the COX-2 inhibitor may be administered via the gastrointestinal tract (*e.g.,* oral, sublingual, or rectal administration), injection (*e.g.,* subcutaneous, intradermal, intravenous, intraperitoneal, or intramuscular), respiratory administration, or topical administration (*e.g.,* mucosal or cutaneous), preferably via the gastrointestinal tract, such as oral administration.

In some embodiments, the PD-L1 small molecule inhibitor is administered orally, and the COX-2 inhibitor is administered orally.

In some embodiments, the total daily dose of the PD-L1 small molecule inhibitor may range from 30 to 2400 mg; more preferably 500 to 2000 mg; for example, 600 mg, 700 mg, 800 mg, 900 mg, 1000 mg, 1100 mg, 1200 mg, 1300 mg, 1400 mg, 1500 mg, 1600 mg, 1700 mg, 1800 mg, or 1900 mg.

In some embodiments, the administration frequency of the PD-L1 small molecule inhibitor is twice daily, once daily, or once every two days, preferably twice daily.

In some embodiments, the total daily dose of the PD-L1 small molecule inhibitor ranges from 500 to 2000 mg, for example, 600 mg, 700 mg, 800 mg, 900 mg, 1000 mg, 1100 mg, 1200 mg, 1300 mg, 1400 mg, 1500 mg, 1600 mg, 1700 mg, 1800 mg, or 1900 mg; The administration frequency is twice daily, once daily, or once every two days, preferably twice daily (BID).

In some embodiments, the total daily dose of the PD-L1 small molecule inhibitor is 1000 mg, 1100 mg, 1200 mg, 1300 mg, 1400 mg, 1500 mg, 1600 mg, 1700 mg, 1800 mg, or 1900 mg; The administration frequency is twice daily.

In some embodiments, the PD-L1 small molecule inhibitor is administered orally at the total daily dose and frequency described above.

In some embodiments, the PD-L1 small molecule inhibitor is administered orally at a total daily dose of 1000 to 1900 mg and a frequency of twice daily.

In some embodiments, the total daily dose of the COX-2 inhibitor ranges from 50 to 250 mg, preferably 100 to 200 mg, for example, 100 mg or 200 mg.

In some embodiments, the administration frequency of the COX-2 inhibitor is once daily or twice daily.

In some embodiments, the total daily dose of the COX-2 inhibitor is 100 mg, 120 mg, 140 mg, 160 mg, 180 mg, or 200 mg; The administration frequency is once daily or twice daily.

In some embodiments, the COX-2 inhibitor is administered orally at the total daily dose and frequency described above.

In some embodiments, the PD-L1 small molecule inhibitor is administered orally at a total daily dose of 1000 to 1900 mg and a frequency of twice daily, and the COX-2 inhibitor is administered orally at a total daily dose of 100 to 200 mg and a frequency of once or twice daily.

The term "pharmaceutically acceptable salt" refers to a salt of the compound of the present disclosure prepared with relatively non-toxic, pharmaceutically acceptable acids or bases. When the compound of the present disclosure contains relatively acidic functional groups, base addition salts can be obtained by contacting the neutral form of such compounds with a sufficient amount of pharmaceutically acceptable base in a pure solution or suitable inert solvent. When the compound of the present disclosure contains relatively basic functional groups, acid addition salts can be obtained by contacting the neutral form of such compounds with a sufficient amount of pharmaceutically acceptable acid in a pure solution or suitable inert solvent.

The term "pharmaceutical excipient" refers to the excipients and additives used in drug production and prescription preparation; It includes all substances in pharmaceutical preparations other than the active pharmaceutical ingredients. See the Pharmacopoeia of the People's Republic of China (2015 Edition) Part IV, or Handbook of Pharmaceutical Excipients (Raymond C Rowe, 2009 Sixth Edition).

The term "treatment" refers to any one of the following situations: (1) alleviating one or more biological manifestations of a disease; (2) interfering with one or more points in the biological cascade that causes the disease; (3) slowing the progression of one or more biological manifestations of the disease.

The term "therapeutically effective amount" refers to the amount of a compound administered to a subject that is sufficient to effectively treat the disease. The therapeutically effective amount will vary depending on the compound, type of disease, severity of the disease, age of the subject, *etc.,* but can be adjusted by those skilled in the art as needed.

The term "subject" refers to any animal that has received or will receive treatment, preferably a mammal, most preferably a human. Mammals include, but are not limited to, cattle, horses, sheep, pigs, cats, dogs, mice, rats, rabbits, guinea pigs, monkeys, humans, *etc.*

On the basis of conforming to common knowledge in the field, the above preferred conditions can be combined arbitrarily to obtain various preferred embodiments of the present disclosure.

The reagents and raw materials used in the present disclosure are all commercially available.

The positive and progressive effects of the present disclosure are as follows: In the treatment of tumor diseases, the PD-L1 small molecule inhibitor combined with different small molecule drugs (such as COX-2 inhibitors and chemotherapeutic drug molecules) has a synergistic effect, demonstrating better antitumor activity or longer survival time compared to using small molecule drugs alone.

### BRIEF DESCRIPTION OF THE DRAWINGS

FIG. 1 shows the tumor volume growth curves of mice in each group in Example 1.
FIG. 2 shows the tumor growth inhibition curves of mice in each group in Example 1.
FIG. 3 shows the body weight change curves of mice in each group in Example 1.
FIG. 4 shows the relative body weight change rate curves of mice in each group in Example 1.
FIG. 5 shows the tumor volume growth curves of mice in each group in Example 2.
FIG. 6 shows the tumor growth inhibition curves of mice in each group in Example 2.
FIG. 7 shows the body weight change curves of mice in each group in Example 2.
FIG. 8 shows the relative body weight change rate curves of mice in each group in Example 2.
FIG. 9 shows the survival curves of mice in each group in Example 3.
FIG. 10 shows the body weight change curves of mice in each group in Example 3.
FIG. 11 shows the relative body weight change rate curves of mice in each group in Example 3.
FIG. 12 shows the body weight change curves of mice in each group in Example 4.
FIG. 13 shows the relative body weight change rate curves of mice in each group in Example 4.
FIG. 14 shows the fluorescence signal intensity curves of mice in each group in Example 4.
FIG. 15 shows the tumor growth inhibition curves of mice in each group in Example 4.
FIG. 16 shows the survival curves of mice in each group in Example 4.

### DETAILED DESCRIPTION OF THE PREFERRED EMBODIMENT

p.o.: "per os" or "oral"; i.p.: intraperitoneal; i.v.: intravenous; BID: twice daily; QD: once daily; Q2D: once every two days; BIW: twice weekly; QW: once weekly.

### Example 1: Pharmacodynamic Study of Test Drugs on Mouse Breast Cancer 4T1 hPD-L1 Model

### 1. Experimental Materials

Experimental animals: 100 BALB/c mice, female, 6 to 8 weeks old, weighing 18 to 21 g, purchased from Shanghai Jihui Laboratory Animal Care Co., Ltd. (SCXK (Shanghai) 2020-0009; Certificate No.: 20220009001922).

Drugs: Compound of Formula I 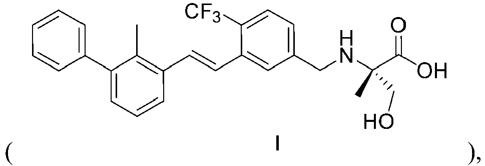 manufacturer: Pharmaron (Tianjin) Process Development and Manufacturing Co., Ltd., batch number: TJ-MAX-10181-A-2; Capecitabine, manufacturer: Shanghai Yuanye Bio-Technology Co., Ltd., batch number: J10J12L137020.

### 2. Experimental Procedures and Methods

### 2.1 Cell Culture

Murine 4T1 hPD-L1 cells were grown adherently *in vitro* under culture conditions of RPMI-1640 medium supplemented with 10% heat-inactivated fetal bovine serum and 4 µg/mL Blasticidin (Beyotime/ST018-1mL), at 37°C with 5% CO₂. Cells were passaged 2 to 3 times per week. When cells were in the exponential growth phase, the cells were harvested, counted, and inoculated subcutaneously on the right dorsal side of BALB/c mice.

### 2.2 Tumor Cell Inoculation and Group Administration

100 µL of 1 × 10⁶ 4T1 hPD-L1 cell suspension was inoculated subcutaneously on the right dorsal side of BALB/c mice. On the 6th day after inoculation, suitable animals were selected based on tumor volume and body weight, and stratified randomization was used to divide them into 4 groups, with 10 mice per group. Administration began on the day of grouping.

**Table 1: Animal Experiment Grouping and Administration Regimen**

| **Group** | **n** | **Treatment Drug** | **Dose** | **Administration Volume** | **Administration Route** | **Administration Frequency** |
|---|---|---|---|---|---|---|
| 1 | 10 | Vehicle Control (Solvent Control) | -- | 10 µL/g | p.o. | BID |
| 2 | 10 | Compound of Formula I | 120 mg/kg | 10 µL/g | p.o. | BID |
| 3 | 10 | Capecitabine | 100 mg/kg | 10 µL/g | p.o. | QD |
| 4 | 10 | Compound of Formula I + Capecitabine | 120 mg/kg + 100 mg/kg | 10 µL/g + 10 µL/g | p.o. + p.o. | BID + QD |

### 2.3 Preparation of Test Drugs

**Table 2: Preparation Methods of Test Drugs**

| **Compound** | **Preparation Method** | **Concentration (mg/mL)** | **Storage Conditions** |
|---|---|---|---|
| (20% SBE-β-CD + 10% Kolliphor RH40) | 100.00 mL of sterile water was pipetted into a volumetric flask. Magnetic stirring was initiated to create a vortex in the liquid surface. 40.00 g of SBE-β-CD was slowly added into the vortex using a medicinal spoon, followed by the addition of 20.00 g of Kolliphor RH40. The mixture was continuously stirred until clear, then diluted to 200.00 mL to obtain an aqueous solution containing 20% SBE-β-CD + 10% Kolliphor RH40. | -- | 2 to 8°C |
| 0.5% CMC-Na | 100.00 mL of sterile water was pipetted into a volumetric flask. Magnetic stirring was initiated to create a vortex in the liquid surface. 1.00 g of CMC-Na was slowly added into the vortex using a medicinal spoon. The mixture was continuously stirred until clear, then diluted to 200.00 mL to obtain an aqueous solution containing 0.5% CMC-Na. | -- | |
| Compound of Formula I | 242.67 mg of the compound of Formula I was weighed and added to 20.00 mL of an aqueous solution containing 20% SBE-β-CD + 10% Kolliphor RH40. The mixture was vortexed for 1 to 2 minutes to ensure thorough mixing, followed by sonication for 15 minutes to obtain a suspension with a concentration of 12 mg/mL. | 12 | 2 to 8°C protected from light |
| Capecitabine | 101.01 mg of the compound Capecitabine was weighed and added to 10.00 mL of an aqueous solution containing 0.5% CMC-Na. The mixture was vortexed for 1 to 2 minutes to ensure thorough mixing, followed by sonication for 15 minutes to obtain a clear solution with a concentration of 6 mg/mL. | 10 | |

### 2.4 Experimental indicators

The experimental indicators were examined to determine whether tumor growth could be inhibited, delayed, or cured. Tumors were measured three times per week using a digital vernier caliper. The tumor volume was calculated using the formula: V = 0.5a × b², where a and b represent the long and short diameters of the tumor, respectively. The tumor growth inhibition efficacy of compounds was calculated as: TGI (%) = [1 - (TVᵢ - TV₀) / (TV_{Vi} - TV_{V0})] × 100% (where TV₀ represents the mean volume of the treatment group on day 0, TV_{V0} represents the mean volume of the control group on day 0; TVᵢ represents the mean volume of the treatment group on day i, and TV_{Vi} represents the mean volume of the control group on day i).

### 2.5 Experiment Termination

If the animals' health condition continued to deteriorate, or the tumor volume exceeded 2,000 mm³, or severe illness or pain occurred, euthanasia was required. In the following circumstances, the veterinarian was notified and euthanasia was performed:
(1) Significant emaciation with body weight loss exceeding 20%;
(2) Inability to freely access food and water;
(3) The experiment was terminated when the average tumor volume in the control group reached 2000 mm³.

### 2.6 Data Analysis

GraphPad Prism 8.0 software was used for plotting. Changes in tumor volume in mice were analyzed by Two-way ANOVA and compared with the vehicle control group using Dunnett's method, with P < 0.05 considered statistically significant.

### 3. Experimental Results

### 3.1 Tumor Volume

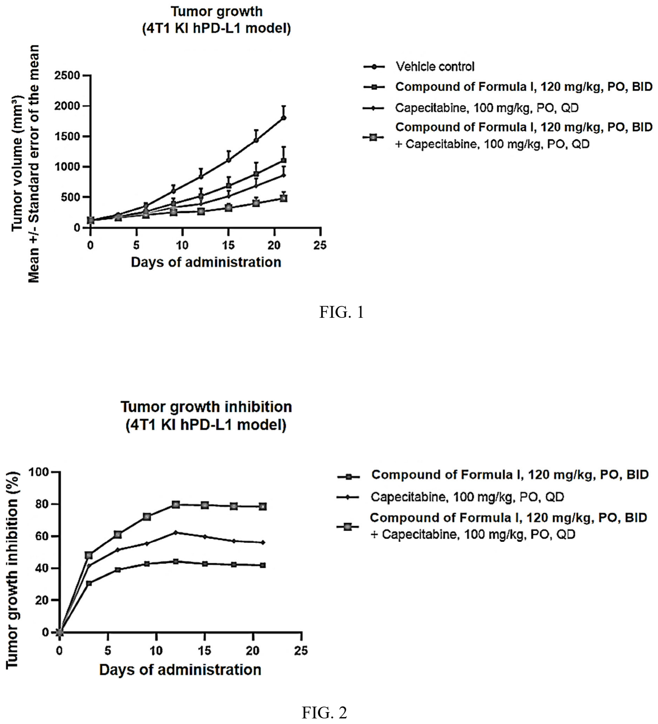

The tumor volumes, tumor growth inhibition effects, and statistical analysis results of each treatment group in the pharmacodynamic experiment of the test drug on the 4T1 hPD-L1 mouse breast cancer model are shown in Table 3, FIG. 1, and FIG. 2.

**Table 3: Tumor Volume (mm³) (Mean)**

| **Group** | **Tumor Volume at the Beginning of the Experiment/Day 0 (mm³)** | **Tumor Volume at the End of the Experiment/Day 21 (mm³)** | **TGI (%)/Day 21** |
|---|---|---|---|
| 1 | 125.38 | 1807.62 | / |
| 2 | 125.52 | 1103.30 | 41.88 |
| 3 | 125.17 | 862.73 | 56.16 |
| 4 | 125.22 | 484.07 | 78.67 |

The mean tumor volume of the compound of Formula I (Group 2) was 1103.30 mm³ on Day 21 after p.o. administration, showing a significant difference compared to the control group (Group 1, tumor volume of 1807.62 mm³ on Day 21) (TGI% = 41.88%).

The mean tumor volume of Capecitabine (Group 3) was 862.73 mm³ on Day 21 after p.o. administration, showing a significant difference compared to the control group (Group 1, tumor volume of 1807.62 mm³ on Day 21) (TGI% = 56.16%).

The mean tumor volume of the combination therapy group of the compound of Formula I and Capecitabine (Group 4) was 484.07 mm³ on Day 21 after the administration, showing a more pronounced significant difference compared to the control group (Group 1, tumor volume of 1807.62 mm³ on Day 21) (TGI% = 78.67%). Compared to the monotherapy groups of the compound of Formula I (Group 2) and Capecitabine (Group 3), the combination therapy group showed significantly enhanced tumor growth inhibition effects with synergistic effects.

### 3.2 Mouse Body Weight

**Table 4: Changes in Body Weight of Treated Mice (Mean)**

| **Group** | **Number of Animals/ (Initial/Final)** | **Body Weight of Animals at the Beginning of the Experiment (g)** | **Body Weight of Animals at the End of the Experiment (g)** | **Relative Body Weight Change of Animals at the End of the Experiment (%)** |
|---|---|---|---|---|
| 1 | 10/10 | 24.64 | 26.97 | 9.46 |
| 2 | 10/10 | 24.20 | 25.55 | 5.66 |
| 3 | 10/10 | 24.27 | 25.49 | 5.07 |
| 4 | 10/10 | 24.15 | 24.95 | 3.40 |

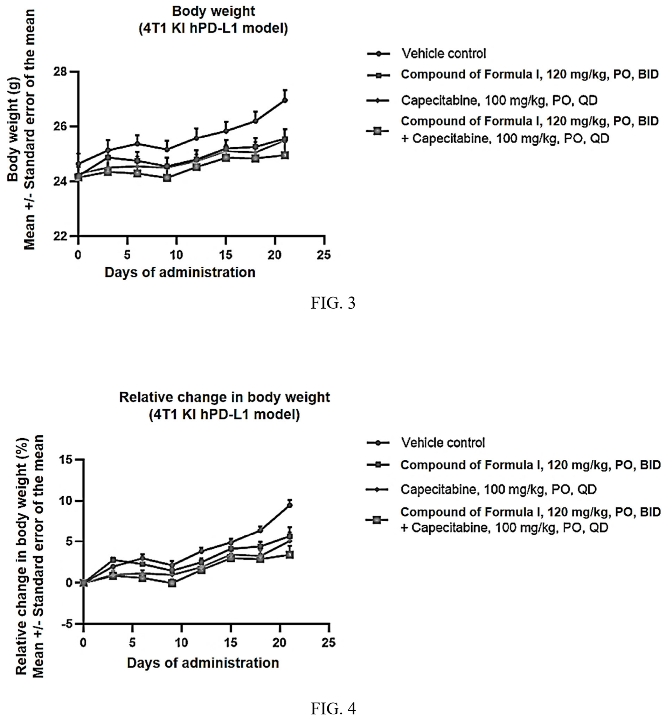

The results of body weight and relative body weight changes of mice in each treatment group are shown in Table 4, FIG. 3, and FIG. 4. During this experiment, no significant body weight loss or other abnormal symptoms were observed in any treatment group of mice, indicating good tolerance to all treatments in mice.

### Example 2: Pharmacodynamic Study of Test Drugs on Mouse Breast Cancer 4T1 hPD-L1 Model

### 1. Experimental Materials

Experimental animals: 100 BALB/c mice, female, 6 to 8 weeks old, weighing 18 to 21 g, purchased from:
Shanghai Jihui Laboratory Animal Care Co., Ltd. (SCXK (Shanghai) 2020-0009; Certificate No.: 20220009001922).

Drugs: Compound of Formula I, manufacturer: Pharmaron (Tianjin) Process Development and Manufacturing Co., Ltd., batch number: TJ-MAX-10181-A-2; Celecoxib, manufacturer: Shanghai Yuanye Bio-Technology Co., Ltd., batch number: D11A6S2152.

### 2. Experimental Procedures and Methods

### 2.1 Cell Culture

Murine 4T1 hPD-L1 cells were grown adherently *in vitro* under culture conditions of RPMI-1640 medium supplemented with 10% heat-inactivated fetal bovine serum and 4 µg/mL Blasticidin (Beyotime/ST018-1mL), at 37°C with 5% CO₂. Cells were passaged 2 to 3 times per week. When cells were in the exponential growth phase, the cells were harvested, counted, and inoculated subcutaneously on the right dorsal side of BALB/c mice.

### 2.2 Tumor Cell Inoculation and Group Administration

100 µL of 1 × 10⁶ 4T1 hPD-L1 cell suspension was inoculated subcutaneously on the right dorsal side of BALB/c mice. On the seventh day after inoculation, stratified randomization was used to divide into 4 groups (10 mice per group) based on tumor volume and body weight. Administration began on the day of grouping.

**Table 5: Animal Experiment Grouping and Administration Regimen**

| **Group** | **n** | **Treatment Drug** | **Dose** | **Administration Volume** | **Administration Route** | **Administration Frequency** |
|---|---|---|---|---|---|---|
| 1 | 10 | Vehicle Control (Solvent Control) | -- | 10 µL/g | p.o. | BID |
| 2 | 10 | Compound of Formula I | 120 mg/kg | 10 µL/g | p.o. | BID |
| 3 | 10 | Celecoxib | 60 mg/kg | 10 µL/g | p.o. | BID |
| 4 | 10 | Compound of Formula I + Celecoxib | 120 mg/kg + 60 mg/kg | 10 µL/g + 10 µL/g | p.o. + p.o. | BID + BID |

### 2.3 Preparation of Test Drugs

**Table 6: Preparation Methods of Test Drugs**

| **Compound** | **Preparation Method** | **Concentration (mg/mL)** | **Storage Conditions** |
|---|---|---|---|
| (20% SBE-β-CD + 10% Kolliphor RH40) | 100.00 mL of sterile water was pipetted into a volumetric flask. Magnetic stirring was initiated to create a vortex in the liquid surface. 40.00 g of SBE-β-CD was slowly added into the vortex using a medicinal spoon, followed by the addition of 20.00 g of Kolliphor RH40. The mixture was continuously stirred until clear, then diluted to 200.00 mL to obtain an aqueous solution containing 20% SBE-β-CD + 10% Kolliphor RH40. | -- | 2 to 8°C |
| 0.5% MC + 0.1% Tween 80 | 100.00 mL of sterile water was pipetted into a volumetric flask. Magnetic stirring was initiated to create a vortex in the liquid surface. 1.00 g of MC was slowly added into the vortex using a medicinal spoon, followed by the addition of 0.20 mL of Tween 80. The mixture was continuously stirred until clear, then diluted to 200.00 mL to obtain an aqueous solution containing 0.5% MC + 0.1% Tween 80. | -- | |
| Compound of Formula I | 546.00 mg of the compound of Formula I was weighed and added to 45.00 mL of an aqueous solution containing 20% SBE-β-CD + 10% Kolliphor RH40. The mixture was vortexed for 1 to 2 minutes to ensure thorough mixing, followed by sonication for 15 minutes to obtain a suspension with a concentration of 12 mg/mL. | 12 | 2 to 8°C protected from light |
| Celecoxib | 146.94 mg of the compound Celecoxib was weighed and added to 24.00 mL of an aqueous solution containing 0.5% MC + 0.1% Tween 80. The mixture was vortexed for 1 to 2 minutes to ensure thorough mixing, followed by sonication for 15 minutes to obtain a suspension with a concentration of 6 mg/mL. | 6 | |

### 2.4 Experimental indicators

The experimental indicators were examined to determine whether tumor growth could be inhibited, delayed, or cured. Tumors were measured three times per week using a digital vernier caliper. The tumor volume was calculated using the formula: V = 0.5a × b², where a and b represent the long and short diameters of the tumor, respectively. The tumor growth inhibition efficacy of compounds was calculated as: TGI (%) = [1 - (TVᵢ - TV₀) / (TV_{Vi} - TV_{V0})] × 100% (where TV₀ represents the mean volume of the treatment group on day 0, TV_{V0} represents the mean volume of the control group on day 0; TVᵢ represents the mean volume of the treatment group on day i, and TV_{Vi} represents the mean volume of the control group on day i).

### 2.5 Experiment Termination

If the animals' health condition continued to deteriorate, or the tumor volume exceeded 2,000 mm³, or severe illness or pain occurred, euthanasia was required. In the following circumstances, the veterinarian was notified and euthanasia was performed:
(1) Significant emaciation with body weight loss exceeding 20%;
(2) Inability to freely access food and water;
(3) The experiment was terminated when the average tumor volume in the control group reached 2000 mm³.

### 2.6 Data Analysis

GraphPad Prism 8.0 software was used for plotting. Changes in tumor volume in mice were analyzed by Two-way ANOVA and compared with the vehicle control group using Dunnett's method, with *P* < 0.05 considered statistically significant.

### 3. Experimental Results

### 3.1 Tumor Volume

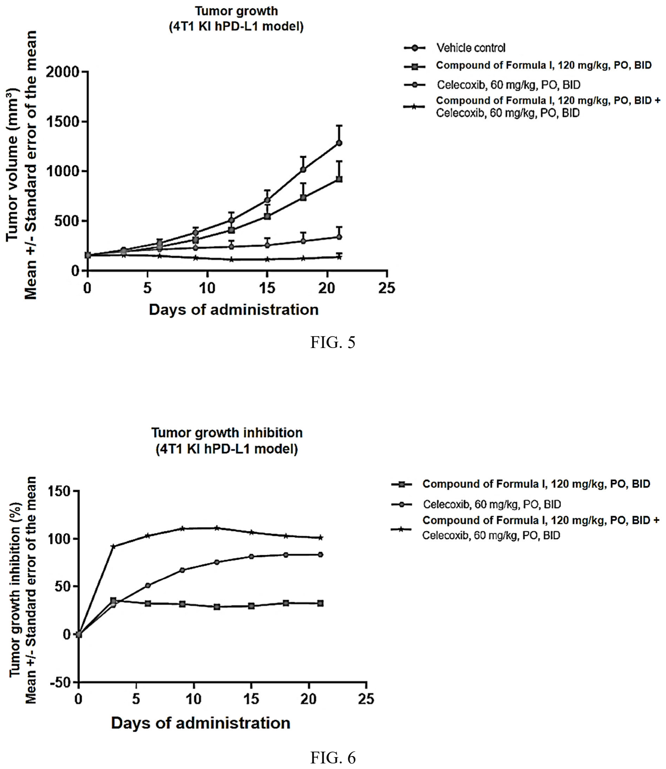

The tumor volumes, tumor growth inhibition effects, and statistical analysis results of each treatment group in the pharmacodynamic experiment of the test drug on the 4T1 hPD-L1 mouse breast cancer model are shown in Table 7, FIG. 5, and FIG. 6.

**Table 7: Tumor Volume (mm³) (Mean)**

| **Group** | **Tumor Volume at the Beginning of the Experiment/Day 0 (mm³)** | **Tumor Volume at the End of the Experiment/Day 21 (mm³)** | **TGI (%)/Day 21** |
|---|---|---|---|
| 1 | 157.14 | 1290.12 | / |
| 2 | 160.33 | 921.91 | 32.78 |
| 3 | 159.34 | 341.70 | 83.90 |
| 4 | 156.99 | 140.02 | 101.50 |

The mean tumor volume of the compound of Formula I (Group 2) was 921.91 mm³ on Day 21 after p.o. administration, showing a significant difference compared to the control group (Group 1, tumor volume of 1290.12 mm³ on Day 21) (TGI% = 32.78%).

The mean tumor volume of the Celecoxib (Group 3) was 341.70 mm³ on Day 21 after p.o. administration, showing a significant difference compared to the control group (Group 1, tumor volume of 1290.12 mm³ on Day 21) (TGI% = 83.90%).

The mean tumor volume of the combination therapy group of the compound of Formula I and Celecoxib (Group 4) was 140.02 mm³ on Day 21 after the administration, showing a more pronounced significant difference compared to the control group (Group 1, tumor volume of 1290.12 mm³ on Day 21) (TGI% = 101.50%). Compared to the monotherapy groups of the compound of Formula I (Group 2) and Celecoxib, the combination therapy group showed significantly enhanced tumor growth inhibition effects.

### 3.2 Mouse Body Weight

**Table 8: Changes in Body Weight of Treated Mice (Mean)**

| **Group** | **Number of Animals/ (Initial/Final)** | **Body Weight of Animals at the Beginning of the Experiment (g)** | **Body Weight of Animals at the End of the Experiment (g)** | **Relative Body Weight Change of Animals at the End of the Experiment (%)** |
|---|---|---|---|---|
| 1 | 10/10 | 22.67 | 24.89 | 9.84 |
| 2 | 10/10 | 23.04 | 24.85 | 8.01 |
| 3 | 10/10 | 22.68 | 23.68 | 4.48 |
| 4 | 10/10 | 22.52 | 23.62 | 4.95 |

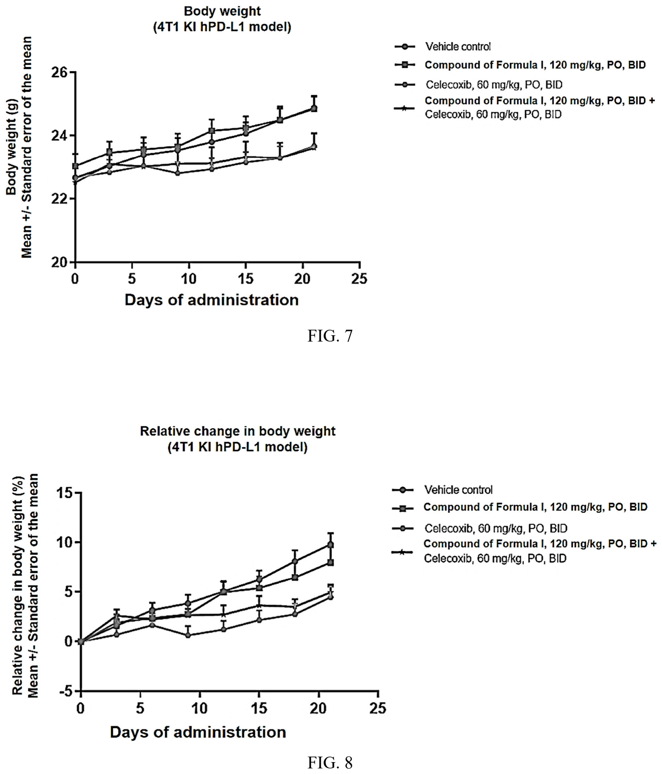

The results of body weight and relative body weight changes of mice in each treatment group are shown in Table 8, FIG. 7, and FIG. 8. During this experiment, no significant body weight loss or other abnormal symptoms were observed in any treatment group of mice, indicating good tolerance to all treatments in mice.

### Example 3: Study on the Growth and Metastasis of Orthotopic Xenograft Tumors and the Prolongation of Mouse Survival Time in a BALB/c Mouse 4T1 Breast Cancer Cell Model Using Test Drugs

### 1. Experimental Materials

Experimental animals: 50 BALB/c mice, female, 6 to 8 weeks old, weighing 18 to 20 g. Zhejiang Vital River Laboratory Animal Technology Co., Ltd., Tongxiang Branch (SCKX (Zhejiang) 2021-0006); Certificate No.: 20220728Abzz0600000694.

Drugs: Compound of Formula I, manufacturer: Pharmaron (Tianjin) Process Development and Manufacturing Co., Ltd., batch number: TJ-MAX-10181-A-2; Paclitaxel (6 mg/mL), manufacturer: Corden Pharma Latina S.P.A., batch number: 2D07013.

### 2. Experimental Procedures and Methods

### 2.1 Cell Culture

Murine 4T1 cells were grown adherently *in vitro* under culture conditions of RPMI-1640 medium supplemented with 10% heat-inactivated fetal bovine serum at 37°C with 5% CO₂. Cells were passaged 2 to 3 times per week. When the cells were in the exponential growth phase, the cells were harvested, counted, and inoculated into the fourth fat pad on the left abdomen of BALB/c mice.

### 2.2 Tumor Cell Inoculation and Group Administration

50 µL of 1 × 10⁵ 4T1 cell suspension was inoculated into the fourth fat pad on the left abdomen of BALB/c mice. On the second day after inoculation, the animals were grouped using stratified randomization based on tumor inoculation sequence and body weight, and administration began on the day of grouping. 13 days after inoculation, orthotopic tumors were surgically resected (administration was paused on the day of surgery).

**Table 9: Animal Experiment Grouping and Administration Regimen**

| **Group** | **Number** | **Treatment Drug** | **Dose (mg/kg)** | **Administration Volume** | **Administration Route** | **Administration Frequency** |
|---|---|---|---|---|---|---|
| 1 | 10 | Vehicle Control | -- | 10 µL/g | PO | BID |
| 2 | 10 | Paclitaxel | 6 | 10 µL/g | IP | Q2D |
| 3 | 10 | Compound of Formula I | 60 | 10 µL/g | PO | BID |
| 4 | 10 | Compound of Formula I + Paclitaxel | 60 + 6 | 10 µL/g | PO + IP | BID + Q2D |

### 2.3 Preparation of Test Drugs

**Table 10: Preparation Methods of Test Drugs**

| **Compound** | **Preparation Method** | **Concentration (mg/mL)** | **Storage Conditions** |
|---|---|---|---|
| (20% SBE-β-CD + 10% Kolliphor RH40) | 100.00 mL of sterile water was pipetted into a volumetric flask. Magnetic stirring was initiated to create a vortex in the liquid surface. 40.00 g of SBE-β-CD was slowly added into the vortex using a medicinal spoon, followed by the addition of 20.00 g of Kolliphor RH40. The mixture was continuously stirred until clear, then diluted to 200.00 mL to obtain an aqueous solution containing 20% SBE-β-CD + 10% Kolliphor RH40. | -- | 2 to 8°C |
| Compound of Formula I | 60.67 mg of the compound of Formula I was weighed and added to 10.00 mL of an aqueous solution containing 20% SBE-β-CD + 10% Kolliphor RH40. The mixture was vortexed for 1 to 2 minutes to ensure thorough mixing, followed by sonication for 15 minutes to obtain a suspension with a concentration of 6 mg/mL. | 6 | 2 to 8°C protected from light |
| Paclitaxel | 0.50 mL of Paclitaxel stock solution (concentration: 6 mg/mL) was pipetted and mixed with 4.5 mL of physiological saline, then vortexed for 1 to 2 minutes to ensure thorough mixing, resulting in a solution with a concentration of 0.60 mg/mL. | 0.60 | 2 to 8°C protected from light |

### 2.4 Experimental indicators

The experimental indicators were examined to determine whether tumor growth could be inhibited, delayed, or cured. Tumors were measured three times per week using a digital vernier caliper. The tumor volume was calculated using the formula: V = 0.5a × b², where a and b represent the long and short diameters of the tumor, respectively. The tumor growth inhibition efficacy of compounds was calculated as: TGI (%) = [1 - (TVᵢ - TV₀) / (TV_{Vi} - TV_{V0})] × 100% (where TV₀ represents the mean volume of the treatment group on day 0, TV_{V0} represents the mean volume of the control group on day 0; TVᵢ represents the mean volume of the treatment group on day i, and TV_{Vi} represents the mean volume of the control group on day i);

### 2.5 Experiment Termination

If the animals' health condition continued to deteriorate, or the tumor volume exceeded 2,000 mm³, or severe illness or pain occurred, euthanasia was required. In the following circumstances, the veterinarian was notified and euthanasia was performed:
(1) Significant emaciation with body weight loss exceeding 20%;
(2) Inability to freely access food and water;
(3) The experiment was terminated when the average tumor volume in the control group reached 2000 mm³.

### 2.6 Data Analysis

GraphPad Prism 8.0 software was used for plotting. Changes in tumor volume in mice were analyzed by Two-way ANOVA and compared with the vehicle control group using Dunnett's method, with *P* < 0.05 considered statistically significant.

### 3. Experimental Results

### 3.1 Survival Curves

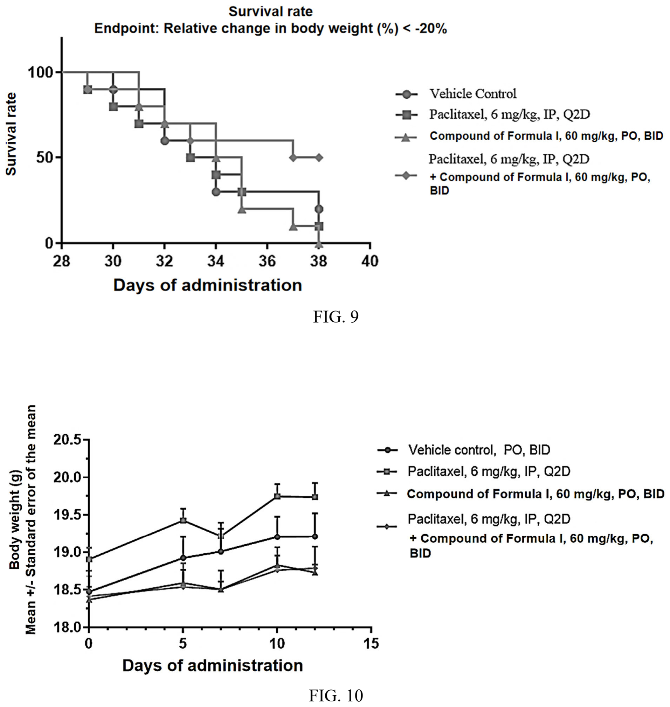

After inoculation with 4T1 cells and corresponding treatments in each group, the combination therapy group of the compound of Formula I with Paclitaxel (Group 4) was compared with the vehicle control group (Group 1), the monotherapy group of the compound of Formula I (Group 3), and the monotherapy group of Paclitaxel (Group 2). The survival rate of mice in the combination therapy group was 50% on day 38, while the survival rate was 0% in the monotherapy group of the compound of Formula I (Group 3) and 10% in the monotherapy group of Paclitaxel (Group 2). The combination therapy group significantly improved the survival rate of mice. See FIG. 9.

### 3.2 Mouse Body Weight

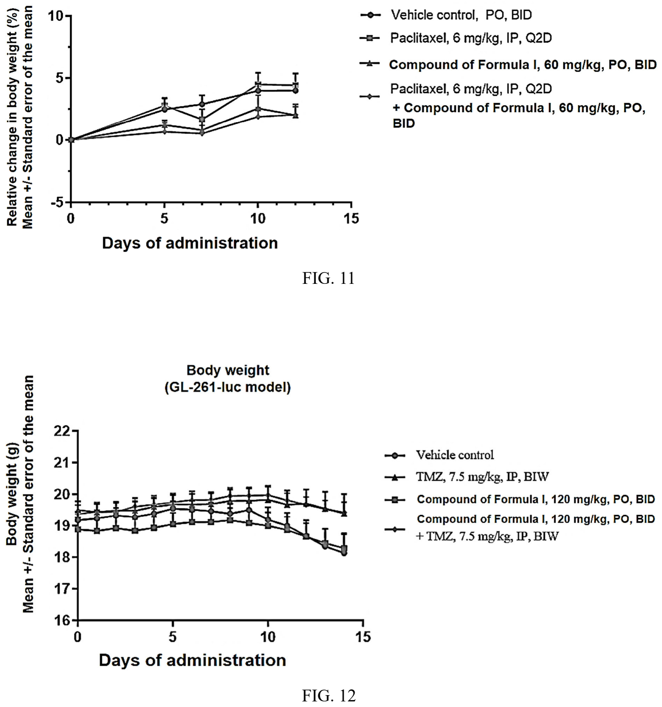

After 12 days of administration, the body weights of mice in all treatment groups remained stable with no significant adverse reactions observed; The body weights of mice returned to normal shortly after tumor resection surgery. The changes in body weight and relative body weight of tumor-bearing mice in each treatment group are shown in FIG. 10 and FIG. 11.

### Example 4: Pharmacodynamic and Survival Time Study of Test Drugs on Mouse Glioma Cell GL-261-luc Brain Orthotopic Xenograft Tumor Model

### 1. Experimental Materials

Experimental animals: 57 C57 BL/6 mice, female, 6 to 8 weeks old, weighing 16 to 20 g, purchased from Shanghai Jihui Laboratory Animal Care Co., Ltd.

Drugs: Compound of Formula I 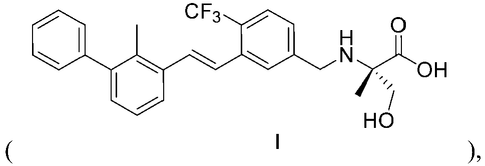 manufacturer: Pharmaron (Tianjin) Process Development and Manufacturing Co., Ltd., batch number: TJ-MAX-10181-A-2; TMZ (Temozolomide), manufacturer: Shanghai Aladdin Biochemical Technology Co., Ltd., batch number: J2227749.

### 2. Experimental Procedures and Methods

### 2.1 Cell Culture

Murine GL-261-luc cells were grown adherently *in vitro* under culture conditions of DMEM medium supplemented with 10% heat-inactivated fetal bovine serum and 0.6 mg/mL G418 (stock concentration: 100 mg/mL), at 37°C with 5% CO₂. Cells were passaged 2 to 3 times per week. When cells were in the exponential growth phase, the cells were harvested, counted, and inoculated into the right caudate nucleus of C57 BL/6 mice.

### 2.2 Tumor Cell Inoculation and Group Administration

GL-261-luc cells in logarithmic growth phase were harvested at full confluence with viability guaranteed 90% or more. Mice were anesthetized via intraperitoneal injection of 41.6 mg/kg Zoletil according to body weight. 1 × 10⁵/2 µL/mouse cells was orthotopically inoculated into the right caudate nucleus of each mouse, and the skin was sutured with 4/0 suture. After recovery, mice were returned to their cages. 4 days after inoculation, mice were imaged and grouped using the IVIS Lumina XR small animal imager. Fluorescence signal intensity was used for stratified randomization into 4 groups (9 mice per group). Administration began on the day of grouping, with daily weight monitoring and observation of mouse growth status. Mice were imaged twice weekly.

**Table 11: Animal Experiment Grouping and Administration Regimen**

| **Group** | **Number** | **Treatment Drug** | **Dose (mg/kg)** | **Administration Volume** | **Administration Route** | **Administration Frequency** |
|---|---|---|---|---|---|---|
| 1 | 9 | Vehicle Control | -- | 10 µL/g | PO | BID |
| 2 | 9 | TMZ | 7.5 | 10 µL/g | IP | BIW |
| 3 | 9 | Compound of Formula I | 120 | 10 µL/g | PO | BID |
| 4 | 9 | Compound of Formula I + TMZ | 120 + 7.5 | 10 µL/g | PO + IP | BID + BIW |

### 2.3 Preparation of Test Drugs

**Table 12: Preparation Methods of Test Drugs**

| **Compound** | **Preparation Method** | **Concentration (mg/mL)** | **Storage Conditions** |
|---|---|---|---|
| (20% SBE-β-CD + 10% Kolliphor RH40) | 100.00 mL of sterile water was pipetted into a volumetric flask. Magnetic stirring was initiated to create a vortex in the liquid surface. 40.00 g of SBE-β-CD was slowly added into the vortex using a medicinal spoon, followed by the addition of 20.00 g of Kolliphor RH40. The mixture was continuously stirred until clear, then diluted to 200.00 mL to obtain an aqueous solution containing 20% SBE-β-CD + 10% Kolliphor RH40. | -- | 2 to 8°C |
| Compound of Formula I | 108.36 mg of the compound of Formula I was weighed and added to 9.03 mL of an aqueous solution containing 20% SBE-β-CD + 10% Kolliphor RH40. The mixture was vortexed for 1 to 2 minutes to ensure thorough mixing, followed by sonication for 10 minutes to obtain a suspension with a concentration of 12.0 mg/mL. | 12 | 2 to 8°C protected from light |
| TMZ | 3.82 mg of compound TMZ was weighed, added to 5.09 mL of physiological saline, vortexed for 1 to 2 minutes to ensure thorough mixing, and sonicated for 1 to 2 minutes to obtain a clear solution with a concentration of 0.75 mg/mL. | 0.75 | |

### 2.4 Experimental indicators

The experimental indicators were examined to determine whether tumor growth could be inhibited, delayed, or cured. According to the experimental design, tumor growth was monitored by imaging mice using the IVIS Lumina XR small animal imager. The tumor growth inhibition efficacy of the compounds was evaluated using TGI (Tumor Growth Inhibition), where TGI (%) = [1 - (TFtreatment-Dn - TFtreatment-D0) / (TFcontrol-Dn - TFcontrol-D0)] × 100% (where TFtreatment-D0 represents the fluorescence signal intensity of the treatment group on day 0, TFcontrol-D0 represents the fluorescence signal intensity of the control group on day 0; TFtreatment-Dn represents the fluorescence signal intensity of the treatment group on day n, and TFcontrol-Dn represents the fluorescence signal intensity of the control group on day n). The calculation formula for an increase in life span (ILS) is: ILS = (MSTTreatment - MSTControl) / MSTControl × 100% (where MSTTreatment is the median survival days for the treatment group, and MSTControl is the median survival days for the control group).

### 2.5 Data Analysis

Tumor size was analyzed using the statistical software GraphPad Prism based on fluorescence signal intensity values. Two-way ANOVA was used to compare whether there were significant differences in fluorescence signal intensity between the control group and each treatment group, with p < 0.05 considered statistically significant and p < 0.01 considered highly statistically significant.

### 3. Experimental Results

### 3.1 Mouse Body Weight

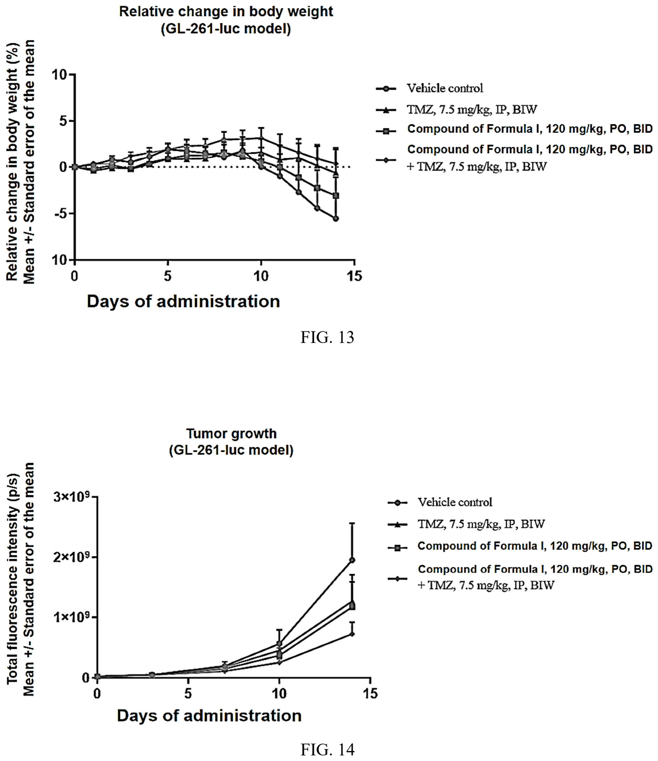

In the pharmacodynamic experiment of the test drug acting on the mouse glioma GL-261-luc brain orthotopic xenograft tumor model, the results of body weight changes and relative body weight changes of each group of tumor-bearing mice are shown in Table 13, FIG. 12, and FIG. 13.

**Table 13: Body Weight Changes of Treated Mice (Mean)**

| **Group** | **Number of Animals/ (Initial/Final)** | **Body Weight of Animals at the Beginning of the Experiment (g)** | **Body Weight of Animals at the End of the Experiment (g)** | **Relative Body Weight Change of Animals at the End of the Experiment (%)** |
|---|---|---|---|---|
| 1 | 9/9 | 19.18 | 18.14 | -5.53 |
| 2 | 9/9 | 19.51 | 19.40 | -0.63 |
| 3 | 9/9 | 18.89 | 18.29 | -3.08 |
| 4 | 9/9 | 19.38 | 19.43 | 0.36 |

Experimental results showed that by day 14 of administration: Vehicle control group (Group 1): 2 mice showed > 10% body weight loss, including 1 mouse with > 15% body weight loss; TMZ group (temozolomide) (Group 2): 2 mice showed > 10% body weight loss; The compound of Formula I group (Group 3): 2 mice showed > 10% body weight loss; The combination therapy group of the compound of Formula I + TMZ (temozolomide) (Group 4): No significant weight loss was observed in mice.

### 3.2 Fluorescence Signal Intensity and Tumor Growth Inhibition

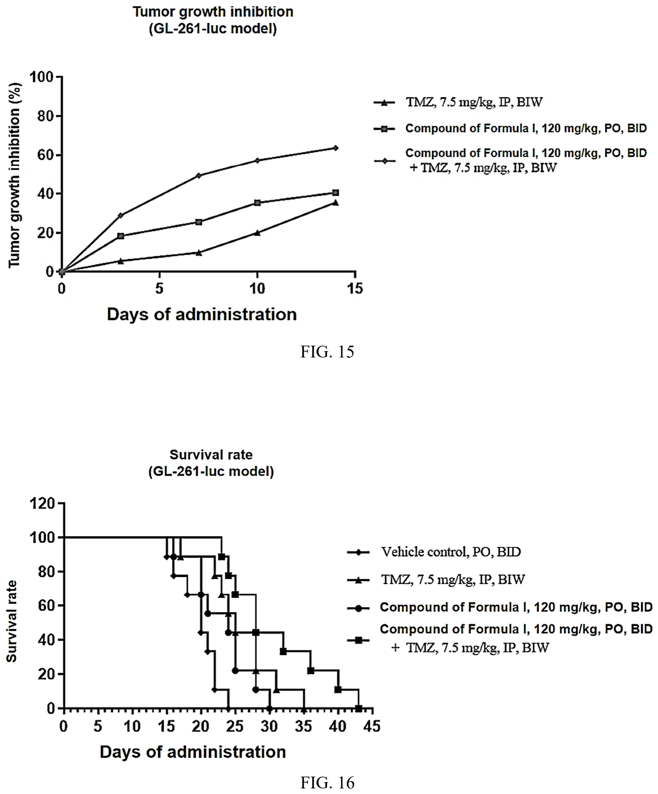

In the pharmacodynamic experiment of the test drug acting on the mouse glioma GL-261-luc model, the changes in fluorescence signal intensity of each group of tumor-bearing mice and the results of the tumor growth inhibition of each group of tumor-bearing mice based on the fluorescence signal intensity are shown in Table 14, FIG. 14, and FIG. 15.

**Table 14: Fluorescence Signal Intensity and Tumor Growth Inhibition of Mice in Each Group**

| **Group** | **Total Fluorescence Intensity at the Beginning of the Experiment/Day 0 (p/s)** | **Total Fluorescence Intensity at the End of the Experiment/Day 14 (p/s)** | **TGI (%)/Day 14** |
|---|---|---|---|
| 1 | 2.79E + 07 | 1.96E + 09 | / |
| 2 | 2.76E + 07 | 1.27E + 09 | 35.51 |
| 3 | 2.75E + 07 | 1.18E + 09 | 40.52 |
| 4 | 2.78E + 07 | 7.31E + 08 | 63.61 |

Experimental results showed that by day 14 of administration, compared with the vehicle control group (Group 1), the tumor growth inhibition rates were 35.51% in the TMZ (temozolomide) group (Group 2), 40.52% in the compound of Formula I group (Group 3), and 63.61% in the combination therapy group of the compound of Formula I + TMZ (temozolomide) (Group 4). Compared with the TMZ (temozolomide) monotherapy group (Group 2) and the compound of Formula I monotherapy group (Group 3), the combination therapy group showed significantly enhanced tumor growth inhibition effects.

### 3.3 Survival Curves

After inoculation with GL-261-luc cells and corresponding treatments, the experiment was terminated until all mice died in each group, survival curves of mice for each group are shown in FIG. 16.

The experimental results showed that by the end of the experiment, compared with the vehicle control group (Group 1), the increase in lifes pan (ILS) was 25%, 20%, and 40% for the TMZ (temozolomide) group (Group 2), the compound of Formula I group (Group 3), and the combination therapy group of the compound of Formula I with TMZ (temozolomide) (Group 4), respectively. The combination therapy group significantly improved the median survival time and the increase in life span of the mice.

## Claims

1. A drug combination A, comprising a PD-L1 small molecule inhibitor and a chemotherapeutic drug molecule.

2. The drug combination A according to claim 1, wherein the drug combination A satisfies one or more of the following conditions:
(1) The drug combination A has a synergistic effect;
(2) The PD-L1 small molecule inhibitor is a compound of Formula I or a pharmaceutically acceptable salt thereof; 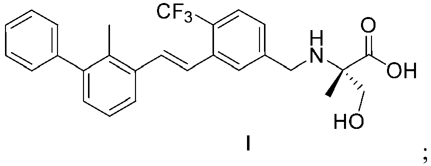
(3) The chemotherapeutic drug molecule is one or more of capecitabine, paclitaxel, oxaliplatin, and temozolomide, preferably capecitabine, paclitaxel, or temozolomide;
(4) The mass ratio of the PD-L1 small molecule inhibitor to the chemotherapeutic drug molecule is (0.04 to 50):1, preferably (0.2 to 16):1, more preferably (0.2 to 2):1 or (6 to 16):1, for example, 0.45:1, 0.58:1, 1.2:1, 10:1, or 11:1;
(5) The active pharmaceutical ingredients of the drug combination A are the PD-L1 small molecule inhibitor and the chemotherapeutic drug molecule;
(6) The PD-L1 small molecule inhibitor is administered via the gastrointestinal tract, injection, respiratory tract, or topical application, such as oral administration;
(7) The chemotherapeutic drug molecule is administered via the gastrointestinal tract, injection, respiratory tract, or topical application, such as oral administration or intravenous injection.

3. The drug combination A according to claim 2, wherein the drug combination A comprises the compound of Formula I and capecitabine, and the mass ratio of the compound of Formula I to capecitabine is preferably (0.2 to 2):1, for example, 0.45:1, 0.58:1, or 1.2:1;
Or, the drug combination A comprises the compound of Formula I and paclitaxel, and the mass ratio of the compound of Formula I to paclitaxel is preferably (6 to 16):1, for example, 10:1 or 11:1;
Or, the drug combination A comprises the compound of Formula I and temozolomide, and the mass ratio of the compound of Formula I to temozolomide is preferably (6 to 16):1, for example, 6:1, 9.5:1, or 16:1.

4. A pharmaceutical composition B, comprising a PD-L1 small molecule inhibitor, a chemotherapeutic drug molecule, and a pharmaceutical excipient.

5. The pharmaceutical composition B according to claim 4, wherein the pharmaceutical composition B satisfies one or more of the following conditions:
(1) The pharmaceutical composition B has a synergistic effect;
(2) The PD-L1 small molecule inhibitor and the chemotherapeutic drug molecule are as described in claim 2;
(3) The content of the PD-L1 small molecule inhibitor is 30 to 500 mg, preferably 40 to 400 mg, for example, 50 mg, 150 mg, or 300 mg;
(4) The content of the chemotherapeutic drug molecule is 10 to 650 mg, preferably 30 to 550 mg, for example, 20 mg, 30 mg, 60 mg, 100 mg, 150 mg, or 500 mg;
(5) The mass ratio of the PD-L1 small molecule inhibitor to the chemotherapeutic drug molecule is (0.04 to 50):1, preferably (0.2 to 16):1, more preferably (0.2 to 2):1 or (6 to 16):1, for example, 0.45:1, 0.58:1, 1.2:1, 10:1, or 11:1;
Preferably, when the pharmaceutical composition B comprises the compound of Formula I according to claim 2, capecitabine, and a pharmaceutical excipient, the mass ratio of the compound of Formula I to capecitabine is preferably (0.2 to 2):1, for example, 0.45:1, 0.58:1, or 1.2:1; When the pharmaceutical composition B comprises the compound of Formula I according to claim 2, paclitaxel, and a pharmaceutical excipient, the mass ratio of the compound of Formula I to paclitaxel is preferably (6 to 16):1, for example, 10:1 or 11:1; When the pharmaceutical composition B comprises the compound of Formula I according to claim 2, temozolomide, and a pharmaceutical excipient, the mass ratio of the compound of Formula I to temozolomide is preferably (6 to 16):1, for example, 6:1, 9.5:1, or 16:1;
(6) The active pharmaceutical ingredients of the pharmaceutical composition B are the PD-L1 small molecule inhibitor and the chemotherapeutic drug molecule;
(7) The dosage form of the pharmaceutical composition B comprises a gastrointestinal dosage form and a parenteral dosage form, preferably a capsule, a tablet, a pill, a granule, a powder, or an oral liquid preparation.

6. A pharmaceutical composition C, comprising:
a first pharmaceutical composition C-1, comprising a PD-L1 small molecule inhibitor and a pharmaceutical excipient; and,
a second pharmaceutical composition C-2, comprising a chemotherapeutic drug molecule and a pharmaceutical excipient.

7. The pharmaceutical composition C according to claim 6, wherein the pharmaceutical composition C satisfies one or more of the following conditions:
(1) The pharmaceutical composition C has a synergistic effect;
(2) The PD-L1 small molecule inhibitor and the chemotherapeutic drug molecule are as described in claim 2;
(3) The content of the PD-L1 small molecule inhibitor is 30 to 500 mg, preferably 40 to 400 mg, for example, 50 mg, 150 mg, or 300 mg;
(4) The content of the chemotherapeutic drug molecule is 10 to 650 mg, preferably 30 to 550 mg, for example, 20 mg, 30 mg, 60 mg, 100 mg, 150 mg, or 500 mg;
(5) The mass ratio of the PD-L1 small molecule inhibitor to the chemotherapeutic drug molecule is (0.04 to 50):1, preferably (0.2 to 16):1, more preferably (0.2 to 2):1 or (6 to 16):1, for example, 0.45:1, 0.58:1, 1.2:1, 10:1, or 11:1;
Preferably, when the first pharmaceutical composition C-1 comprises the compound of Formula I according to claim 2 and a pharmaceutical excipient, and the second pharmaceutical composition C-2 comprises capecitabine and a pharmaceutical excipient, then the mass ratio of the compound of Formula I to capecitabine is preferably (0.2 to 2):1, for example, 0.45:1, 0.58:1, or 1.2:1; When the first pharmaceutical composition C-1 comprises the compound of Formula I according to claim 2 and a pharmaceutical excipient, and the second pharmaceutical composition C-2 comprises paclitaxel and a pharmaceutical excipient, then the mass ratio of the compound of Formula I to paclitaxel is preferably (6 to 16):1, for example, 10:1 or 11:1; When the first pharmaceutical composition C-1 comprises the compound of Formula I as described above and a pharmaceutical excipient, and the second pharmaceutical composition C-2 comprises temozolomide and a pharmaceutical excipient, then the mass ratio of the compound of Formula I to temozolomide is preferably (6 to 16):1, for example, 6:1, 9.5:1, or 16:1;
(6) The pharmaceutical composition C consists of the first pharmaceutical composition C-1 and the second pharmaceutical composition C-2; The first pharmaceutical composition C-1 consists of the PD-L1 small molecule inhibitor and a pharmaceutical excipient; The second pharmaceutical composition C-2 consists of the chemotherapeutic drug molecule and a pharmaceutical excipient;
(7) The first pharmaceutical composition C-1 is presented in an oral dosage form;
(8) The second pharmaceutical composition C-2 is presented in an oral dosage form or an injectable dosage form.

8. A combination kit D, comprising:
a first container, comprising the first pharmaceutical composition C-1 according to claim 6 or 7; and,
a second container, comprising the second pharmaceutical composition C-2 according to claim 6 or 7.

9. A drug combination E, comprising a PD-L1 small molecule inhibitor and a COX-2 inhibitor.

10. The drug combination E according to claim 9, wherein the drug combination E satisfies one or more of the following conditions:
(1) The drug combination E has a synergistic effect;
(2) The PD-L1 small molecule inhibitor is a compound of Formula I or a pharmaceutically acceptable salt thereof; 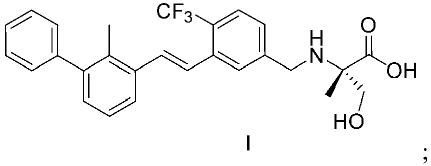
(3) The COX-2 inhibitor is nimesulide, meloxicam, celecoxib, or rofecoxib, preferably celecoxib;
(4) The mass ratio of the PD-L1 small molecule inhibitor to the COX-2 inhibitor is (0.1 to 10):1, more preferably (2 to 10):1, for example, 6:1 or 7.5:1;
(5) The active pharmaceutical ingredients of the drug combination E are the PD-L1 small molecule inhibitor and the COX-2 inhibitor;
(6) The PD-L1 small molecule inhibitor is administered via the gastrointestinal tract, injection, respiratory tract, or topical application, such as oral administration;
(7) The COX-2 inhibitor is administered via the gastrointestinal tract, injection, respiratory tract, or topical application, such as oral administration.

11. A pharmaceutical composition F, comprising a PD-L1 small molecule inhibitor, a COX-2 inhibitor, and a pharmaceutical excipient.

12. The pharmaceutical composition F according to claim 11, wherein the pharmaceutical composition F satisfies one or more of the following conditions:
(1) The pharmaceutical composition F has a synergistic effect;
(2) The PD-L1 small molecule inhibitor and the COX-2 inhibitor are as described in claim 10;
(3) The content of the PD-L1 small molecule inhibitor is 30 to 500 mg, preferably 40 to 400 mg, for example, 50 mg, 150 mg, or 300 mg;
(4) The content of the COX-2 inhibitor is 50 to 250 mg, preferably 100 to 200 mg, for example, 100 mg or 200 mg;
(5) The mass ratio of the PD-L1 small molecule inhibitor to the COX-2 inhibitor is (0.1 to 10):1, preferably (2 to 10):1, for example, 6:1 or 7.5:1;
(6) The active pharmaceutical ingredients of the pharmaceutical composition F are the PD-L1 small molecule inhibitor and the COX-2 inhibitor;
(7) The dosage form of the pharmaceutical composition F comprises a gastrointestinal dosage form and a parenteral dosage form, preferably a capsule, a tablet, a pill, a granule, a powder, or an oral liquid preparation.

13. A pharmaceutical composition G, comprising:
a first pharmaceutical composition G-1, comprising a PD-L1 small molecule inhibitor and a pharmaceutical excipient; and,
a second pharmaceutical composition G-2, comprising a COX-2 inhibitor and a pharmaceutical excipient.

14. The pharmaceutical composition G according to claim 13, wherein the pharmaceutical composition G satisfies one or more of the following conditions:
(1) The pharmaceutical composition G has a synergistic effect;
(2) The PD-L1 small molecule inhibitor and COX-2 inhibitor are as described in claim 10;
(3) The content of the PD-L1 small molecule inhibitor is 30 to 500 mg, preferably 40 to 400 mg, for example, 50 mg, 150 mg, or 300 mg;
(4) The content of the COX-2 inhibitor is 50 to 250 mg, preferably 100 to 200 mg, for example, 100 mg or 200 mg;
(5) The mass ratio of the PD-L1 small molecule inhibitor to the COX-2 inhibitor is (0.1 to 10):1, preferably (2 to 10):1, for example, 6:1 or 7.5:1;
(6) The pharmaceutical composition G consists of the first pharmaceutical composition G-1 and the second pharmaceutical composition G-2; The first pharmaceutical composition G-1 consists of the PD-L1 small molecule inhibitor and a pharmaceutical excipient, and the second pharmaceutical composition G-2 consists of the COX-2 inhibitor and a pharmaceutical excipient;
(7) The first pharmaceutical composition G-1 is presented in an oral dosage form;
(8) The second pharmaceutical composition G-2 is presented in an oral dosage form.

15. A combination kit H, comprising:
a first container, comprising the first pharmaceutical composition G-1 according to claim 13 or 14; and,
a second container, comprising the second pharmaceutical composition G-2 according to claim 13 or 14.

16. Use of the drug combination A according to any one of claims 1 to 3, the pharmaceutical composition B according to claim 4 or 5, the pharmaceutical composition C according to claim 6 or 7, the drug combination E according to claim 9 or 10, the pharmaceutical composition F according to claim 11 or 12, or the pharmaceutical composition G according to claim 13 or 14 in the manufacture of a medicament for treating tumors; The tumor is preferably one or more of gastric cancer, nasopharyngeal carcinoma, lung cancer, colorectal cancer, breast cancer, colon cancer, bladder cancer, cervical cancer, endometrial cancer, pancreatic cancer, prostate cancer, hepatocellular carcinoma, melanoma, head and neck cancer, esophageal cancer, ovarian cancer, bone cancer, central nervous system tumors, adrenal tumors, renal cell carcinoma, neuroblastoma, and glioblastoma, more preferably breast cancer, gastric cancer, nasopharyngeal carcinoma, or glioblastoma.

17. A method of treating tumors, comprising administering a therapeutically effective amount of the drug combination A according to any one of claims 1 to 3, the pharmaceutical composition B according to claim 4 or 5, the pharmaceutical composition C according to claim 6 or 7, the drug combination E according to claim 9 or 10, the pharmaceutical composition F according to claim 11 or 12, or the pharmaceutical composition G according to claim 13 or 14 to a subject in need thereof.

18. The method of treating tumors according to claim 17, wherein the method of treating tumors satisfies one or more of the following conditions:
(1) The tumor is one or more of gastric cancer, nasopharyngeal carcinoma, lung cancer, colorectal cancer, breast cancer, colon cancer, bladder cancer, cervical cancer, endometrial cancer, pancreatic cancer, prostate cancer, hepatocellular carcinoma, melanoma, head and neck cancer, esophageal cancer, ovarian cancer, bone cancer, central nervous system tumors, adrenal tumors, renal cell carcinoma, neuroblastoma, and glioblastoma, preferably breast cancer, gastric cancer, nasopharyngeal carcinoma, or glioblastoma;
(2) The total daily dose of the PD-L1 small molecule inhibitor ranges from 30 to 2400 mg; preferably 500 to 2000 mg; for example, 600 mg, 700 mg, 800 mg, 900 mg, 1000 mg, 1100 mg, 1200 mg, 1300 mg, 1400 mg, 1500 mg, 1600 mg, 1700 mg, 1800 mg, or 1900 mg;
(3) The administration frequency of the PD-L1 small molecule inhibitor is twice daily, once daily, or once every two days, preferably twice daily;
(4) When administering a therapeutically effective amount of the drug combination A according to any one of claims 1 to 3, the pharmaceutical composition B according to claim 4 or 5, or the pharmaceutical composition C according to claim 6 or 7 to a subject in need thereof, and when the chemotherapeutic drug molecule is capecitabine, then the total daily dose ranges from 1000 to 3000 mg/m², preferably 1000 mg/m², 1250 mg/m², 1500 mg/m², 1750 mg/m², 2000 mg/m², 2250 mg/m², 2500 mg/m², 2750 mg/m², or 3000 mg/m², for example, 2000 mg/m² or 2500 mg/m²; The administration frequency is once daily or twice daily, preferably twice daily;
(5) When administering a therapeutically effective amount of the drug combination A according to any one of claims 1 to 3, the pharmaceutical composition B according to claim 4 or 5, or the pharmaceutical composition C according to claim 6 or 7 to a subject in need thereof, and when the chemotherapeutic drug molecule is paclitaxel, then the total daily dose ranges from 50 to 200 mg/m², preferably 50 mg/m², 75 mg/m², 80 mg/m², 100 mg/m², 125 mg/m², 135 mg/m², 145 mg/m², 155 mg/m², 165 mg/m², 175 mg/m², 185 mg/m², 195 mg/m², or 200 mg/m², for example, 80 mg/m², 135 mg/m², or 175 mg/m²; The administration frequency is once every two weeks, once every three weeks, or once every four weeks; preferably once every three weeks;
(6) When administering a therapeutically effective amount of the drug combination A according to any one of claims 1 to 3, the pharmaceutical composition B according to claim 4 or 5, or the pharmaceutical composition C according to claim 6 or 7 to a subject in need thereof, and when the chemotherapeutic drug molecule is temozolomide, then the total daily dose ranges from 100 to 250 mg/m², preferably 100 mg/m², 125 mg/m², 150 mg/m², 175 mg/m², 200 mg/m², 225 mg/m², or 250 mg/m², for example, 150 mg/m² or 200 mg/m²; The administration frequency is once daily for 5 days, followed by 23 days off, then entering the next cycle;
(7) When administering a therapeutically effective amount of the drug combination E according to claim 9 or 10, the pharmaceutical composition F according to claim 11 or 12, or the pharmaceutical composition G according to claim 13 or 14 to a subject in need thereof, then the total daily dose of the COX-2 inhibitor ranges from 50 to 250 mg, preferably 100 to 200 mg, for example, 100 mg or 200 mg;
(8) When administering a therapeutically effective amount of the drug combination E according to claim 9 or 10, the pharmaceutical composition F according to claim 11 or 12, or the pharmaceutical composition G according to claim 13 or 14 to a subject in need thereof, then the administration frequency of the COX-2 inhibitor is once daily or twice daily.
